Europäisches Patentamt
European Patent Office
Office européen des brevets

(19) (11) Numéro de publication: **0 208 634 B1**

(12) **FASCICULE DE BREVET EUROPEEN**

(45) Date de publication du fascicule du brevet: **06.09.89**

(51) Int. Cl.⁴: **C 07 D 207/448**, C 08 F 283/00, C 08 G 77/26, C 08 L 83/08

(21) Numéro de dépôt: **86420173.6**

(22) Date de dépôt: **27.06.86**

ERRATUM

(SEITE, SPALTE, ZEILE)
(PAGE, COLUMN, LINE)
(PAGE, COLONNE, LIGNE)

| DIE TEXTSTELLE : TEXT PUBLISHED : LE PASSAGE SUIVANT : | | | | LAUTET BERICHTIGT: SHOULD READ : DEVRAIT ETRE LU : |
|---|---|---|---|---|
| 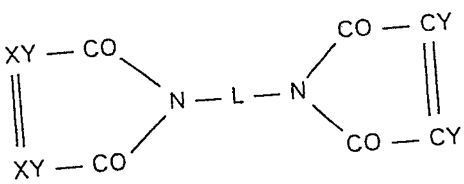 | 4 | 5 | 38-43 | 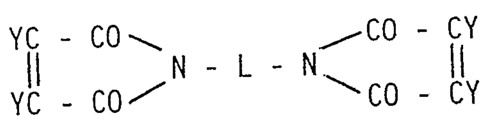 |
| | 14 | 25 | 53-58 | |
| | 16 | 29 | 56-61 | |
| | 18 | 34 | 1-6 | |

Tag der Entscheidung über die Berichtigung / Date of decision on rectification: / Date de décision portant sur modification: 09.11.89

Ausgabe- und Veröffentlichungstag: / Issue and publication date: / Date d'edition et de publication: 03.01.90

Patbl.Nr) EPB no:) Bull. no:) 90/(

(19) Europäisches Patentamt
European Patent Office
Office européen des brevets

(11) Numéro de publication: **0 208 634 B1**

(12) **FASCICULE DE BREVET EUROPEEN**

(45) Date de publication du fascicule du brevet: **06.09.89**

(51) Int. Cl.⁴: **C 07 D 207/448,** C 08 F 283/00, C 08 G 77/26, C 08 L 83/08

(21) Numéro de dépôt: **86420173.6**

(22) Date de dépôt: **27.06.86**

(54) **Nouveaux maléimides et nouvelles compositions thermodurcissables les contenant.**

(30) Priorité: **01.07.85 FR 8510190**
**17.04.86 FR 8605744**

(43) Date de publication de la demande: **14.01.87 Bulletin 87/3**

(45) Mention de la délivrance du brevet: **06.09.89 Bulletin 89/36**

(84) Etats contractants désignés: **BE CH DE FR GB IT LI LU NL SE**

(56) Documents cités:
**CHEMICAL ABSTRACTS, vol. 71, no. 1, 7 juillet 1969, page 290, résumé no. 3164h, Columbus, Ohio, US; & SU-A-230 128 (IRKUTSK INSTITUTE OF ORGANIC CHEMISTRY) 30-10-1968**
**CHEMICAL ABSTRACTS, vol. 79, no. 13, 1 octobre 1973, page 455, résumé no. 78311c, Columbus, Ohio, US; G.G. SKVORTSOVA et al.: "Vinyl ethers of imidophenols", & DOKL. VSES. KONF. KHIM. ATSETILENA, 4th 1972, 2, 394-9**
**CHEMICAL ABSTRACTS, vol. 92, no. 1, 7 janvier 1980, page 581, résumé no. 6180e, Columbus, Ohio, US; G.G. SKVORTSOVA et al.: "Reaction of acrylidenevinyloxyanilines with anhydrides of unsaturated dicarboxylic acids", & IZV. SIB. OTD. AKAD. NAUK SSSR, SER. KHIM. NAUK 1979, (4), 129-34**

(73) Titulaire: **RHONE-POULENC CHIMIE, 25, quai Paul Doumer, F-92408 Courbevoie Cédex (FR)**

(72) Inventeur: **Rakoutz, Michel, décédé (FR)**

(74) Mandataire: **Trolliet, Maurice et al, RHONE-POULENC INTERSERVICES Service Brevets Chimie Centre de Recherches de Saint-Fons B.P. 62, F-69192 Saint-Fons Cédex (FR)**

**EP 0 208 634 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne de nouveaux composés comportant un groupement maléimide.

Elle concerne également de nouvelles compositions thermodurcissables à base de composés à groupements imides possédant des propriétés mécaniques améliorées.

Les maléimides N-substitués sont une famille connue de composés chimiques et les bis-maléimides N,N'-disubstitués sont notamment utilisés pour la préparation de polymères thermodurcissables, les polybis-maléimides.

Des monomaléimides sont également connus. Ainsi le brevet américain No. 2 444 536 décrit un procédé de préparation de N-arylmaléimides.

Certains monomaléimides peuvent être utilisés en agrochimie comme insecticides ou fungicides. D'autres peuvent servir à préparer des polymères réticulables sous l'influence de la lumière.

Les monomaléimides peuvent également être employés en mélanges avec des bis-maléimides pour la production de polymères thermodurcissables.

Un des objets de la présente invention consiste en de nouveaux monomaléimides de formule générale (I):

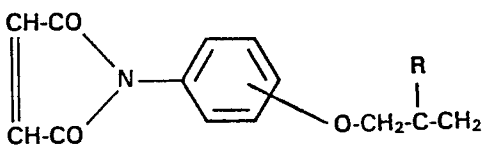

(I)

dans laquelle R représente un atome d'hydrogène ou un radical méthyle.

Ces nouveaux monomaléimides sont:

- le N-(allyloxy-2 phényl)maléimide,
- le N-(allyloxy-3 phényl)maléimide,
- le N-(allyloxy-4 phényl)maléimide,
- le N-(méthallyloxy-2 phényl)maléimide,
- le N-(méthallyloxy-3 phényl)maléimide,
- le N-(méthallyloxy-4 phényl)maléimide.

Les maléimides de formule (I) peuvent être notamment préparés à partir des aminophénols (ortho, méta ou para), selon la réaction de Claisen.

On peut par exemple faire réagir l'aminophénol, dont on a préalablement bloqué la fonction amine par réaction avec l'anhydride acétique pour former l'acétamidophénol, avec selon le cas un halogénure d'allyle (le plus souvent le bromure) ou de méthallyle en solution dans l'acétone et en présence de carbonate dipotassique. La fonction amine est ensuite régénérée par hydrolyse.

On prépare ensuite de manière classique le maléimide correspondant en faisant réagir en solution l'allyloxyaniline ou la méthallyloxyaniline obtenue précédemment avec l'anhydride maléique, en présence d'anhydride acétique, de triéthylamine et d'un sel de nickel (acétate de nickel en particulier).

On obtient ainsi le N-allyloxyphényl-maléimide ou le N-méthallyloxyphényl-maléimide.

Le N-(allyloxy-4 phényl)maléimide est un solide de couleur jaune moutarde ayant un point de fusion de 103°C environ.

L'analyse RMN est en accord avec la structure suivante:

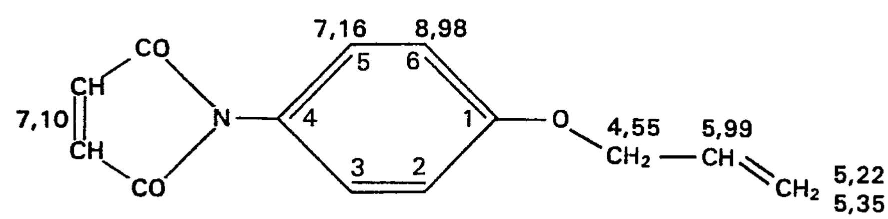

RMN 1H; solvant: DMSO d6; référence: hexaméthyldisiloxane (HMDS)

δ 7,16 (2H, m): H 3,5;

δ 7,19 (2H, s): maléimido;

δ 6,98 (2H, m): H 2,6;

δ 5,99 (1H, M): -CH=;

δ 5,35 et 5,22 (2H, dd): $=CH_2$;

δ 4,55 (2H, d): $OCH_2$.

Le N-(allyloxy-3 phényl)maléimide est un liquide jaune orange visqueux, qui cristallise lentement à température ambiante et qui bout à 150°C environ sous une pression 20 Pa.

L'analyse RMN est en accord avec la structure suivante:

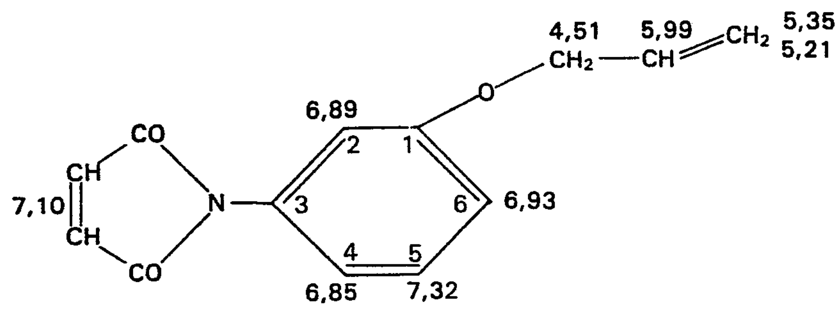

RMN 1H; solvant: DMSO d6; référence: HMDS
δ 6,85, 6,89 et 6,93 (3H, m): H4, H2 et H6;
δ 7,10 (2H, s): maléimido;
δ 7,32 /1H, t): H5;
δ 5,99 (1H, m): -CH=;
δ 5,35 et 5,21 (2H, dd): $=CH_2$;
δ 4,51 (2H, d): $OCH_2$.

Le N-(allyloxy-2 phényl)maléimide est un solide cristallisé jaune clair, ayant un point de fusion d'environ 82°C et un point d'ébullition de 148°C à 155°C sous une pression de 20 Pa.

L'analyse RMN est en accord avec la structure suivante:

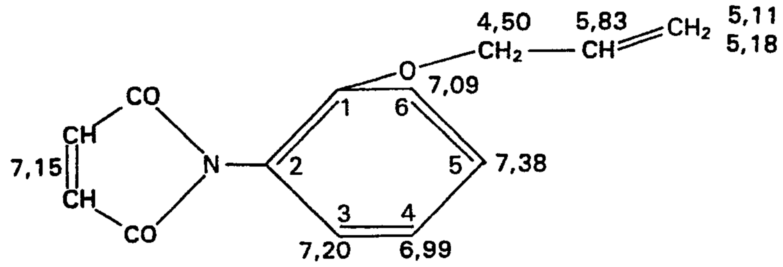

RMN 1H; solvant: DMSO d6; référence: HMDS
δ 7,38 (1H, dt): H5;
δ 7,20 (1H, dd): H3;
δ 7,15 (2H, s): maléimido;
δ 7,09 (1H, dd): H6;
δ 6,99 (1H, dt): H4;
δ 5,83 (1H, m): -CH=;
δ 5,18 et 5,11 (2H, dd): $=CH_2$;
δ 4,50 (2H, d): $OCH_2$.

Le N-(méthallyloxy-4 phényl) maléimide et un solide de couleur beige ayant un point de fusion de 64°C.

L'analyse RMN est en accord avec la structure suivante:

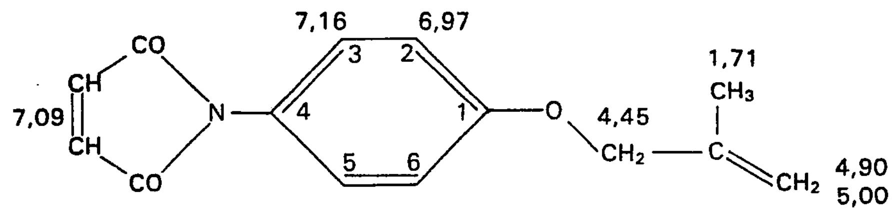

RMN 1H; solvant: DMSO d6; référence: HMDS
δ 7,16 (2H, d): H 3,5;
δ 7,09 (2H, s): maléimido;
δ 6,97 (2H, d): H 2,6;
δ 4,90 et 5,00 (1H, s): $CH_2=$;
δ 4,45 (2H, s): $OCH_2$;
δ 1,71 (3H, s): $CH_3$.

Le N-(méthallyloxy-3 phényl) maléimide est un solide de couleur beige ayant un point de fusion de 39°C.

L'analyse RMN est en accord avec la structure suivante:

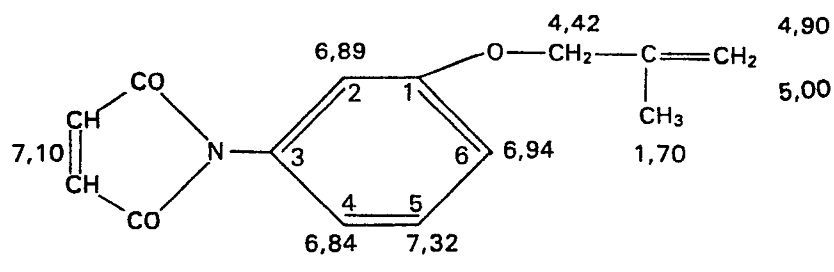

RMN 1H; solvant: DMSO d6; référence: HMDS
δ 7,32 (1H, t): H5;
δ 7,10 (2H, s): maléimido;
δ 6,94 (1H, d): H6;
δ 6,89 (1H, s): H2;
δ 6,84 (1H, d): H4;
δ 4,90 et 5,00 (1H, 1): $CH_2=$;
δ 4,42 (2H, s): $OCH_2$;
δ 1,70 (3H, s): $CH_3$.

Le N-(méthallyloxy-2 phényl) maléimide est un solide de couleur beige ayant un point de fusion de 96°C.

L'analyse RMN est en accord avec la structure suivante:

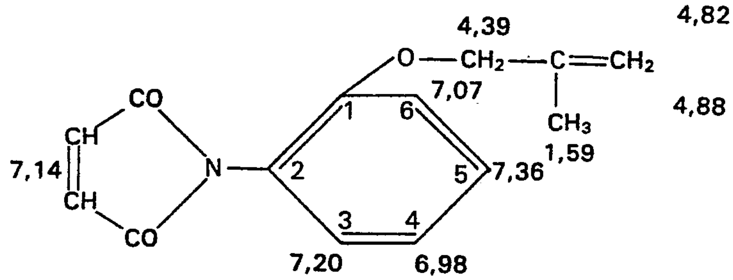

RMN 1H; solvant: DMSO d6; référence: HMDS
δ 7,36 (1H, t): H5;
δ 7,20 (1H, d): H3;
δ 7,14 (2H, s): maléimido;
δ 7,07 (1H, d): H6;
δ 6,98 (1H, t): H4;
δ 4,82 et 4,88 (1H, s): $CH_2=$;
δ 4,39 (2H, s): $OCH_2$;
δ 1,59 (3H, s): $CH_3$.

Les nouveaux monomaléimides de formule générale (I), lorsqu'ils sont utilisés avec un ou plusieurs bis-maléimides pour préparer des compositions thermodurcissables pour moulage ou pour imprégnation, apportent à ces compositions des propriétés mécaniques améliorées par rapport à des compositions préparées sans ces nouveaux monomaléimides.

Plus précisément, un autre objet de la présente invention consiste en de nouvelles compositions thermodurcissables, caractérisées en ce qu'elles sont constituées essentiellement par:

A) un prépolymère obtenu par réaction entre 50°C et 300°C de:
a) un bis-imide ou une association de plusieurs bis-imides de formule générale (II):

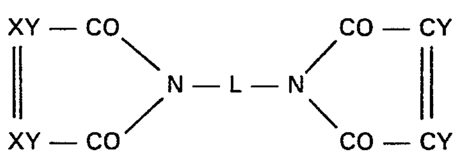
(II)

dans laquelle:

— Y représente un atome d'hydrogène ou un groupement méthyle;

— L représente un radicale divalent tel qu'un radical cyclohexylène; un radical phénylène; le radical méthyl-4 phénylène-1,3; le radical méthyl-2 phénylène-1,3; le radical méthyl-5- phénylène-1,3; le radical diéthyl-2,5 méthyl-3 phénylène-1,4 et les radicaux de formule (III):

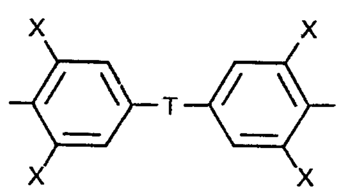
(III)

dans laquelle:
. T représente un lien valentiel simple ou un atome ou groupement suivant:

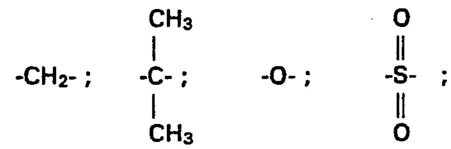

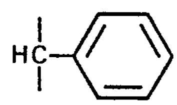 ; 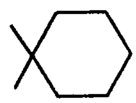

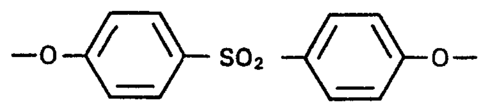

. X représente un atome d'hydrogène, un radical méthyle, éthyle ou isopropyle;
avec
b) un ou plusieurs monomaléimides de formule générale (I):

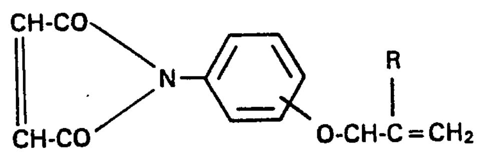
(I)

dans laquelle R représente un atome d'hydrogène ou un radical méthyle;
et éventuellement avec
c) un composé organosilicique comportant dans sa molécule au moins un groupement hydroxyle lié à un atome de silicium;
et B) de l'imidazole ou un dérivé de l'imidazole.

Le bis-maléimide de formule (II) peut être choisi par exemple parmi:
— le N,N'-métaphénylène-bis-maléimide,
— le N,N'-paraphénylène-bis-maléimide,
— le N,N'-4,4'-diphénylméthane-bis-maléimide,
— le N,N'-4,4'-diphényléther-bis-maléimide,
— le N,N'-4,4'-diphénylsulfone-bis-maléimide,
— le N,N'-1,4-cyclohexylène-bis-maléimide,
— le N,N',4,4'-(diphényl-1,1-cyclohexylidène)-bis-maléimide,
— le N,N'-4,4'-(diphényl-2,2 propane)bis-maléimide,
— le N,N'-4,4'-triphénylméthane-bis-maléimide,
— le N,N'-1,3-méthyl-4 phénylène-bis-maléimide.
— le N,N'-1,3-méthyl-2-phénylène-bis maléimide.

Parmi ces bis-maléimides on préfère plus particilièrement utiliser le N,N'-4,4'-diphénylméthane bismaléimide, le N,N'-1,3-méthyl-4 phénylène bismaléimide, le N,N'-1,3-méthyl-2 phénylène bismaléimide et leurs mélanges.

Ces bis-maléimides peuvent être préparés selon les procédés décrits dans le brevet américain No. 3 018 290 et le brevet britannique No. 1 137 290.

Le monomaléimide de formule générale (I) utilisé est choisi parmi:

— le N-(allyloxy-2 phényl)maléimide,
— le N-(allyloxy-3 phényl)maléimide,
— le N-(allyloxy-4 phényl)maléimide,
— le N-(méthallyloxy-2 phényl)maléimide,
— le N-(méthallyloxy-3 phényl)maléimide,
— le N-(méthallyloxy-4 phényl)maléimide,

et leurs mélanges.

Les composés organosiliciques hydroxylés qui entrent à titre facultatif dans le cadre de l'invention sont des composés connus répondant à la formule générale (IV) suivante:

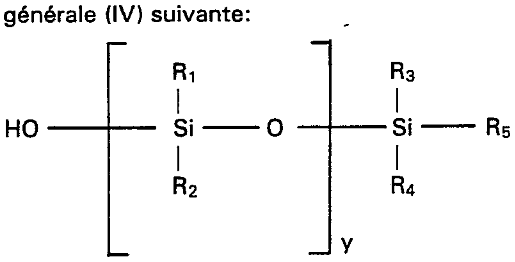
(IV)

dans laquelle $R_1$, $R_2$, $R_3$, $R_4$ et $R_5$, identiques ou différents, représentent:

— un groupement hydroxyle ou un groupement du type $-OR_6$ dans laquelle $R_6$ peut être un radical alkyle, linéaire ou ramifié, ayant de 1 à 6 atomes de carbone ou un radical phényle;
— un atome d'hydrogène;
— un radical alkyle, linéaire ou ramifié, ayant de 1 à 6 atomes de carbone et pouvant être éventuellement substitué par un ou plusieurs atomes de chlore ou de fluor ou par un groupement -CN;
— un radical alcényle, linéaire ou ramifié, ayant de 2 à 6 atomes de carbone;
— un radical phényle, éventuellement substitué par un ou plusieurs radicaux alkyles et/ou alcoxyles ayant de 1 à 4 atomes de carbone, ou par un ou plusieurs atomes de chlore; et y est un nombre entier ou fractionnaire, de 0 à 1000.

Pour un composé organosilicique défini de formule (IV), y est en réalité toujours un nombre entier, mais comme il s'agit en l'occurrence de composés à structure polymérique (lorsque y est supérieur à 1), on a rarement un seul composé, mais le plus souvent un mélange de composés de même structure chimique, qui diffèrent par le nombre d'unités récurrentes de leur molécule; cela conduit à une valeur moyenne de y, qui peut être entière ou fractionnaire.

On peut caractériser les composés organosiliciques hydroxylés du type précité par le rapport du poids des groupements hydroxyle qu'ils possèdent au poids total de leur molécule.

La mise en œuvre d'un composé organosilicique hydroxylé est une mesure permettant notamment de faciliter, lors de la préparation des compositions thermodurcissables selon la présente invention, le passage à l'état fondu des composés à groupements maléimides et de donner aussi à le résine thermodurcissable obtenue une plus grande fluidité à l'état fondu.

Quand on choisit d'utiliser des composés organosiliciques, les composés auxquels on fait appel de préférence pour exécuter la présente invention, sont les composés précités pour lesquels le rapport pondéral des groupements hydroxyle dans la molécule est au moins égal à 0,05% et de préférence à 0,1%.

Parmi les composés organosiliciques appartenant à ce groupe préféré, ceux qui conviennent tout particulièrement bien sont les composés de formule (IV) dans laquelle:

— $R_1$, $R_2$, $R_3$ et $R_4$, identiques ou différents, représentent un radical alkyle, linéaire ou ramifié, ayant de 1 à 6 atomes de carbone ou un radical alcényle, linéaire ou ramifié, ayant de 2 à 6 atomes de carbone ou un radical phényle;
— $R_5$ représente un groupement hydroxyle;
— y est un nombre, entier ou fractionnaire, de 0 à 250.

Il s'agit donc de silane-diols lorsque y est égal à 0 ou bien de polysiloxane-diols lorsque y est différent de 0.

Pour leur préparation on peut se reporter à l'ouvrage de W. NOLL «Chemistry and Technology of Silicones» (traduction anglaise de l'Edition allemande de 1968) édité par Academic Press de New-York.

Les composés organosiliciques qui conviennent tout particulièrement bien sont choisis dans le groupe constitué par:

— le diéthylsilane-diol
— le diphénylsilane-diol
— le méthylphénylsilane-diol
— le tétraméthyl-1,1,3,3 disiloxane-diol-1,3
— le diméthyl-1,1 diphényl-3,3 disiloxane-diol-1,3
— le diméthyl-1,3 diphényl-1,3 disiloxane-diol-1,3
— l'hexaméthyl-1,1,3,3,5,5 trisiloxane-diol-1,5
— l'octaméthyl-1,1,3,3,5,5,7,7 tétrasiloxane-diol-1,7
— le décaméthyl-1,1,3,3,5,5,7,7,9,9 pentasiloxane-diol-1,9
— le dodécaméthyl-1,1,3,3,5,5,7,7,9,9,11,11 hexasiloxane-diol-1,11
— le pentaméthyl-1,3,5,7,9 pentaphényl-1,3,5,7,9 pentasiloxane-diol-1,9

ainsi que leurs homologues supérieures correspondants.

Les composés organosiliciques hydroxylés convenant tout particulièrement bien peuvent également être des mélanges de deux ou plusieurs des composés précités. C'est ainsi que l'on peut utiliser par commodité des huiles polysiloxaniques hydroxylées du commerce. Ce sont en particulier des huiles polyméthylpolysiloxaniques- α, ω dihydroxylées ayant de 0,2 à 0,3% en poids de groupements hydroxyle (huile de Rhône-Poulenc 48 V 500), ou 10 à 12% en poids de groupements hydroxyle (huile de Rhône-Poulenc 48 V 50) ou des huiles ou résines méthylphénylpolysiloxaniques- α, ω dihydroxylées ayant 4,5% à 5% en poids de groupements hydroxyle (huile 50606 de Rhône-Poulenc) ou de 7,5

à 8,5% en poids de groupements hydroxyle (résine 50305 de Rhône-Poulenc); ces huiles ou résines du commerce sont données à titre d'exemple, mais il en existe d'autres pouvant convenir tout aussi bien.

Dans les prépolymères A) préparés à partir d'un ou plusieurs bis-imides de formule (II) et d'un ou de plusieurs monomaléimides de formule (I), on choisit les quantités de réactifs de manière à avoir, en poids par rapport au poids total de ces différents constituants:

— de 50 à 95% de bis-imide(s),
— et de 5 à 50% de monomaléimide(s).

Dans les prépolymères A) préparés à partir d'un ou de plusieurs bis-imides de formule (II), d'un ou de plusieurs monomaléimides de formule (I) et d'un composé organosilicique hydroxylé de formule (IV), on choisit les quantités de réactifs de manière à avoir, en poids par rapport au poids total de ces différents constituants:

— de 40 à 90% de bis-imide(s),
— de 5 à 40% de monomaléimide(s),
— et de 5 à 40% de composé organosilicique hydroxylé.

Pour obtenir des compositions selon l'invention de ce type présentant des propriétés en flexion à chaud plus élevées, il est préférable de mettre une proportion de composé organosilicique de 5 à 20% en poids du poids total de bis-imide(s), monomaléimide(s) et composé organosilicique hydroxylé.

Le dérivé imidazole B) répond à la formule générale (V):

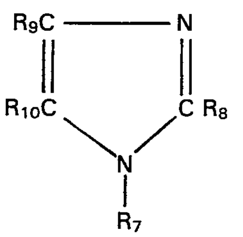

(V)

dans laquelle $R_7$, $R_8$, $R_9$ et $R_{10}$, identiques ou différents, représentent: un atome d'hydrogène, un radicale alkyle ou alcoxy ayant de 1 à 20 atomes de carbone, un radical vinyle, un radical phényle, un groupement nitro, $R_9$ pouvant former avec $R_{10}$ et les atomes de carbone auxquels sont liés ces radicaux un cycle unique comme par exemple un cycle benzénique, $R_7$ pouvant également représenter un groupement carbonyle lié à un 2ème cycle imidazole.

Comme exemples spécifiques de dérivés imidazole, on peut citer, en particulier, l'imidazole ou glyoxaline, le méthyl-1 imidazole, le méthyl-2 imidazole, le diméthyl-1,2 imidazole, le vinyl-1 imidazole, le vinyl-1 méthyl-2 imidazole, le benzimidazole, le carbonyldiimidazole.

Le dérivé imidazole est utilisé en quantités catalytiques. Selon la nature du dérivé imidazole et selon la vitesse de polymérisation souhaitée au stade de la mise en œuvre, on utilise le dérivé imidazole à un taux compris entre 0,02 et 1 % en poids par rapport au prépolymère A).

De préférence on utilise un taux d'imidazole de 0,05 à 0,5% en poids par rapport au prépolymère A).

Les compositions selon l'invention peuvent également contenir une N,N',N''-tris(hydroxyalkyl)hexahydrotriazine; on utilise plus particulièrement la N,N',N''-tris(hydroxyéthyl)hexahydrotriazine-1,3,5, la N,N',N''-tris(hydroxypropyl)hexahydrotriazine-1,3,5 et la N,N',N''-tris(hydroxybutyl)hexahydrotriazine-1,3,5.

On emploiera préférentiellement la N,N',N''-tris(hydroxyéthyl)-hexahydrotriazine-1,3,5 disponible dans le commerce.

Lorsqu'elle est présente la N'N', N''-tris(hydroxyalkyl)hexahydroxytriazine confère aux compositions thermodurcissables une pégosité plus élevée ainsi qu'une amélioration des propriétés thermomécaniques, notamment de la résistance à la flexion.

On utilise habituellement de 0 à 5% en poids de N,N',N''-tris(hydroxyalkyl)hexahydrotriazine par rapport au prépolymère A).

Pour obtenir une bonne efficacité, il est préférable d'utiliser à 0,5 à 2% en poids de N,N',N''-tris(hydroxyalkyl)hexahydrotriazine par rapport au prépolymère A).

Les compositions selon l'invention conduisent après cuisson è des propriétés mécaniques, notamment en flexion, à température ambiante et à chaud (250°C généralement) plus élevées que celles que l'on obtient avec des compositions antérieures, telles que celles décrites dans la demande de brevet français No. 83/17.218 publié sous le numéro 2 553 780.

Divers adjuvants peuvent être incorporés dans les compositions selon l'invention. Ces adjuvants habituellement utilisés et bien connus de l'homme de l'art peuvent être par exemple des stabilisants ou des inhibiteurs de dégradation, les lubrifiants ou des agents de démoulage, des colorants ou des pigments, des charges pulvérulentes ou en particules comme des silicates, des carbonates, le kaolin, la craie, le quartz pulvérisé, le mica ou des microbilles de verre, etc... On peut aussi incorporer des adjuvants modifiant la structure physique du produit obtenu comme par exemple des agents porogènes ou des renforçants fibreux: fibrilles de carbone, de polyimide, de polyamides aromatiques, whiskers, etc...

Le procédé de fabrication est tel que la résine thermodurcissable prête à l'emploi présente suffisamment de souplesse et de collant en couche mince. En outre pour obtenir un matériau homogène après stratification, les réactions génératrices de composés très volatils aux températures de cuisson doivent être peu importantes. Dans ce but, lorsque les réactifs de départ comprennent un silanediol, il est souhaitable de réaliser d'abord la plus grande partie de la réaction d'oligomérisation donnant de l'eau comme sous-produit; cette eau peut être éliminée plus facilement en cours de fabrication de la résine.

Tout d'abord on réalise un mélange intime des composés à groupements maléimides et éventuellement du composé organosilicique hydroxylé. Pour éviter une homopolymérisation prématurée des maléimides qui conduirait à une résine trop visqueuse, le mélange maléimides + éventuellement composé organosilicique hydroxylé est fondu en absence de catalyseur à une température au plus égale à la température de fusion du maléimide le plus difficile à liquéfier. Si le mélange réactionnel comprend un composé organosilicique riche an groupements hydroxyles, le mélange est dans ce cas maintenu fondu de façon à réaliser une partie de l'oligomérisa-

tion du silanediol; de préférence on chauffera ce composé à environ 150°C jusqu'à ce que 40% environ des groupements hydroxyles initiaux aient disparu lors de l'oligomérisation de ce composé. On pourra, à titre de variante, réaliser cette oligomérisation avant introduction des composés à groupements maléimides.

Le dérivé imidazole B) et le cas échéant la N,N',N''-tris(hydroxyalkyl)hexahydrotriazine sont ajoutés ensuite au mélange bien agité, de façon à permettre leur dispersion rapide.

Lorsque le catalyseur est particulièrement actif, il est souhaitable, afin d'éviter son encapsulation dans le réseau polymérique qu'il engendre, de l'ajouter avec un solvant compatible avec le milieu réactionnel. On peut ainsi utiliser un solvant tel que le triallylisocyanurate, le phtalate de diallyle ou le benzoate d'allyle.

On peut également employer un solvant volatil qui sera ultérieurement éliminé par vaporisation sous pression. En effet le mélange est dégazé afin d'éliminer les produits volatils qui sont contre-indiqués pour la préparation des stratifiés. Le mélange est coulé immédiatement après l'homogénéisation.

Les compositions thermodurcissables selon l'invention possèdent une pégosité suffisante pour les applications telles que les stratifiés et les matériaux composites.

Les compositions peuvent être mises en œuvre par des opérations de moulage ou d'imprégnation. Elles peuvent être utilisées pour la réalisation de revêtements, de collages, de stratifiés et matériaux composites renforcés. Le matériau de renforcement peut être sous forme de nappes tissées ou non tissées, d'éléments unidirectionnels ou de fibres coupées naturelles ou synthétiques telles que les filaments ou fibres de verre, de bore, de carbone, de tungstène, de silicium, de polyamide-imides ou polyamides aromatiques. Les compositions présentent un intérêt tout particulier pour l'obtention d'articles intermédiaires préimprégnés sans solvant. L'imprégnation du matériaux fibreux peut être effectuée par application des techniques usuelles telles que l'immersion, l'enduction au racle ou au rideau ou l'imprégnation par transfert. Le film transférable et les articles préimprégnés peuvent être utilisés directement ou bien être emmagasinés en vue d'un emploi ultérieur; ils conservent de manière très satisfaisantes leurs propriétés au cours d'un stockage au froid entre 0 et 10°C.

Les matériaux imprégnés sont utilisables pour la réalisation de pièces ayant des formes et des fonctions variées dans de nombreuses industries comme par exemple dans l'aéronautique. Ces pièces qui peuvent être des pièces de révolution sont obtenues par placage de plusieurs couches de préimprégnés sur une forme ou un support.

On effectue ensuite la réticulation dans les conditions technologiques habituelles relatives aux matériaux composites et en particulier à des températures comprises entre 100 et 300°C.

On peut utiliser aussi les préimprégnés comme renforts ou moyens de réparation de pièces détériorées.

Mais il est aussi possible de concevoir des pièces selon les techniques de l'enroulement filamentaire avec ou sans support, ou de l'injection moulage ou de la pultrusion.

On peut ainsi obtenir des produits conformés à haute résistance mécanique et thermique.

Les exemples suivants sont donnés à titre illustratif de l'invention:

*Example 1*

*Préparation du N-(allyloxy-4 phényl)maléimide:*

Ce composé a été préparé à partir de la para-allyloxyaniline dont un mode d'obtention est décrit dans Journal of American Chemical Society, *44*, pages 1741 à 1744 (1922).

Dans un réacteur en verre muni d'une agitation centrale, d'un thermomètre et d'un réfrigérant ascendant, maintenu à 50°C sous agitation et dans lequel circule un léger courant d'azote, on introduit simultanément en 20 minutes, à l'aide de deux ampoules de coulée:

— 249 g d'une solution acétonique contenant 149,0 g de para-allyloxyaniline;

— 249 g d'une solution acétonique contenant 112,7 g d'anhydride maléique.

La réaction est exothermique et conduit à la formation immédiate d'une suspension jaunâtre.

Lorsque les coulées sont terminées, chaque ampoule est rincée par 10 $cm^3$ d'acétone, qui sont ensuite rajoutés à la masse réactionnelle toujours maintenu sous agitation.

Dans l'ampoule ayant contenu l'anhydride maléique, on charge 163,2 g d'anhydride acétique et dans l'autre ampoule on charge 45,4 g de triéthylamine.

Ces deux composés sont alors coulés en 5 minutes dans le réacteur, puis on ajoute 1,9 $cm^3$ d'une solution aqueuse contenant 0,0528 mole d'acétate de nickel pour 100 $cm^3$ de solution.

Le mélange réactionnel est maintenu à reflux sous agitation pendant 2 h 30 min. La température est ensuite abaissée à 30°C, on rajoute 1349 g d'eau distillée, puis on refroidit le mélange à 15°C sous agitation.

Le précité jaune foncé est essoré, lavé par 100 $cm^3$ d'un mélange 80/20 en volume acétone/eau préalablement refroidi à 10°C, puis par 100 $cm^3$ d'eau distillée.

On sèche le solide essoré pendant 15 h à 40°C sous pression réduite (30 Pa).

On obtient ainsi 215,5 g d'un produit pulvérulent, de couleur jaune moutarde, ayant un point de fusion de 103°C.

Le spectre RMN est en accord avec la structure du N-(allyloxy-4 phényl)maléimide.

*Exemple 2*

*Préparation du N-(allyloxy-3 phényl)maléimide:*

On utilise le même appareillage que dans l'exemple 1 et on suit le même mode opératoire.

Le produit de départ est la métaallyloxyaniline dont un mode de préparation est décrit dans Chemical Abstracts, *51*, 4423 a à g (1957).

Les charges utilisées sont doublées par rapport à celles de l'exemple 1:

— 498 g d'une solution acétonique contenant 298,0 g de métaallyloxyaniline;

— 498 g d'une solution acétonique contenant 225,4 g d'anhydride maléique;

— rinçage de chaque ampoule de coulée par 20 $cm^3$ d'acétone;

— 326,4 g d'anhydride acétique;

— 90,8 g de triéthylamine;

— 3,8 $cm^3$ de solution aqueuse à 0,0528 mole d'acétate de nickel pour 100 $cm^3$ de solution;

— 2698 g d'eau distillée.

L'addition des 2698 g d'eau distillée au mélange réactionnel conduit à la décantation d'une huile de couleur foncée, que l'on extrait par trois fois 250 $cm^3$ d'acétate d'éthyle. Les couches organiques obtenues sont rassemblées et séchées sur du sulfate de sodium.

Après élimination à sec du solvant sous pression réduite (d'abord sous 3000 Pa environ, puis sous 70 Pa environ), on obtient 464 g d'une huile épaisse très foncée titrant 0,309 double liaison éthylénique pour 100 g.

On prélève 22,77 g de ce produit brut auxquels on ajoute 0,2 g d'hydroquinone. On distille sous 12 Pa, dans un réacteur de 50 $cm^3$ muni d'une colonne Vigreux et d'un séparateur de fractions.

On recueille 14,7 g de fraction passant entre 150°C et 155°C sous 20 Pa.

C'est un liquide visqueux, limpide, jaune-orangé, présentant un spectre de RMN en accord avec la structure N-(allyloxy-3 phényl)maléimide.

*Exemple 3*

*Préparation du N-(allyloxy-2 phényl)maléimide:*

Ce composé a été préparé à partir de l'allyloxy-2 aniline dont un mode d'obtention est décrit dans Journal of American Chemical Society, *70*, page 593 (1948).

Dans un réacteur en verre muni d'une agitation centrale, d'un thermomètre et d'un réfrigérant ascendant, maintenu à 50°C sous agitation et dans lequel circule un léger courant d'azote, on introduit simultanément en 30 minutes, à l'aide de 2 ampoules de coulée:

— 443 $cm^3$ d'une solution acétonique contenant 298 g d'allyloxy-2 aniline;

— 443 $cm^3$ d'une solution acétonique contenant 235,2 g d'anhydride maléique.

La réaction est exothermique et conduit à la formation immédiate d'une suspension jaunâtre.

Lorsque les coulées sont terminées, chaque ampoule est rincée par 10 $cm^3$ d'acétone, qui sont ensuite rajoutés à la masse réactionnelle toujours maintenue sous agitation.

Dans l'ampoule ayant contenu l'anhydride maléique, on charge 265,2 g d'anhydride acétique, et dans l'autre ampoule, on charge 60,6 g de triéthylamine.

Ces deux composés sont alors coulés en 6 minutes dans le réacteur, puis on ajoute 3,8 $cm^3$ d'une solution aqueuse contenant 0,0528 mole d'acétate de nickel pour 100 $cm^3$ de solution.

Le mélange réactionnel est maintenu à reflux sous agitation pendant 2 h 30 minutes. La température est ensuite abaissée à 30°C, on rajoute 2500 g d'eau distillée, puis on refroidit le mélange à 20°C sous agitation.

Une huile noire décante à la surface de la phase aqueuse. On siphonne la phase aqueuse et on reprend la phase huileuse par 400 $cm^3$ d'acétate d'éthyle. La phase organique est lavée par deux fois 500 $cm^3$ d'eau distillée jusqu'à pH des eaux de lavage égal à 6. La phase organique décantée est séchée sur 150 g de sulfate de sodium anhydre.

Après élimination à sec du solvant sous pression réduite (d'abord sous 3000 Pa environ, puis sous 70 Pa environ), on obtient 450 g d'une huile épaisse très foncée, que l'on traite par 560 $cm^3$ d'alcool éthylique, le mélange est refroidi à 5°C dans de la glace, et l'on filtre. On obtient 416 g d'un précipité beige, humide. On sèche pendant 15 heures à 40°C sous pression réduite (30 Pa) et on obtient 365 g de produit cristallin, beige, ayant un point de fusion de 82°C.

Le spectre RMN est en accord avec la structure du N-(allyloxy-2 phényl)maléimide.

*Exemple 4*

*Préparation du N-(méthallyloxy-4 phényl)maléimide:*

Ce composé a été préparé à partir de la méthallyloxy-4 aniline.

4.1. *Méthallyloxy-4 aniline:*

Ce composé a été préparé à partir du paraméthallyloxyacétamido benzène. Ce dernier a été obtenu par analogie à la méthode de préparation de l'ortho-acétamidophényl allyl éther décrite dans Journal of American Chemical Society, *70*, page 593 (1948), en remplaçant toutefois le bromure d'allyle par le chlorure de méthallyle, et en opérant en présence d'une quantité catalytique d'iodure de potassium (10% molaire par rapport au chlorure de méthallyle).

Dans un réacteur en verre muni d'une agitation centrale, d'un thermomètre et d'un réfrigérant ascendant, on introduit:

— 205 g de para-méthallyloxyacétamidobenzène

— 400 ml de soude éthanolique 5N on homogénéise, et on ajoute:

— 13,6 ml d'imidazole, soit 20% molaire par rapport au para-méthallyloxyacétamido-benzène.

Le mélange réactionnel est maintenu au reflux, sous agitation, pendant 3 heures.

Le mélange réactionnel est refroidi à 20°C.

On élimine l'éthanol sous pression réduite (70 Pa. environ).

On ajoute dans la masse réactionnelle 250 $cm^3$ d'acétate d'éthyle, et on lave par deux fois 250 $cm^3$ d'eau distillée pour arriver à pH = 7 dans les eaux de lavage.

On élimine l'acétate d'éthyle de la phase organique sous pression réduite (70 Pa. environ) et on obtient 165,5 g de produit brut.

On obtient 135,4 g de produit purifié par distillation, à 113°C sous 0,05 mmHg.

Le spectre RMN est en accord avec la structure de la méthallyloxy-4 aniline.

4.2. *N-(méthallyloxy-4 phényl)maléimide:*

Dans un réacteur en verre muni d'une agitation centrale, d'un thermomètre et d'un réfrigérant ascendant, maintenu à 50°C sous agitation et dans lequel circule un léger courant d'azote, on introduit simultanément, en 15 minutes à l'aide de 2 ampoules de coulée:

— 52 $cm^3$ d'une solution acétonique, contenant 32,6 g de méthallyloxy-4 aniline,

— 52 $cm^3$ d'une solution acétonique, contenant 22,5 g d'anhydride maléique.

La réaction est exothermique et conduit à la formation immédiate d'une suspension jaunâtre.

Lorsque les coulées sont terminées, chaque ampoule est rincée par 10 $cm^3$ d'acétone, qui sont ensuite rajoutés à la masse réactionnelle toujours maintenue sous agitation.

Dans l'ampoule de coulée ayant contenu l'anhydride maléique, on charge 26,5 g d'anhydride acétique et dans l'autre ampoule, on charge 6,1 g de triéthylamine.

Ces deux composés sont alors coulés en 5 minutes dans le réacteur, puis on ajoute 0,4 $cm^3$ d'une solution aqueuse contenant 0,0528 mole d'acétate de nickel pour 100 $cm^3$ de solution.

Le mélange réactionnel est maintenu à reflux sous agitation pendant 2 heures 30 minutes. La température est ensuite abaissée à 20°C.

Le mélange réactionnel est précipité par coulée lente dans 500 $cm^3$ d'eau glacée, sous violente agitation.

Le précipité est filtré, relavé à l'eau distillée refroidie, et séché pendant 15 heures à 40°C sous pression réduite (30 Pa). On obtient 39,4 g de précipité beige, ayant un point de fusion de 64°C.

Le spectre RMN est en accord avec la structure du N-(méthallyloxy-4 phényl)maléimide.

*Exemple 5*

*Préparation du N-(méthallyloxy-3 phényl)maléimide:*

Ce composé a été préparé à partir de la méthallyloxy-3 aniline.

5.1. *Méthallyloxy-3 aniline:*

Le produit de départ est le méta-méthallyloxyacétamidobenzène, dont les conditions d'obtention sont les mêmes que celles décrites dans l'exemple 4, paragraphe 4.1. pour le para-méthallyloxyacétamidobenzène.

Dans un réacteur en verre muni d'une agitation centrale, d'un thermomètre et d'un réfrigérant ascendant, on introduit:

— 205 g de méta-méthallyloxyacétamidobenzène

— 400 ml de soude éthanolique 5N

on homogénéise, et on ajoute:

— 13,6 g d'imidazole, soit 20% molaire.

Le mélange réactionnel est maintenu au reflux, sous agitation, pendant 3 heures.

Le mélange réactionnel est refroidi à 20°C.

On élimine l'éthanol sous pression réduite (70 Pa. environ).

On ajoute dans la masse réactionnelle 250 $cm^3$ d'acétate d'éthyle, et on lave par deux fois 250 $cm^3$ d'eau distillée, pour arriver à pH = 7 dans les eaux de lavage.

On élimine l'acétate d'éthyle de la phase organique sous pression réduite (70 Pa. environ) et on obtient 144 g de produit brut.

On obtient 124 g de produit purifié par destillation, à 85°C sous 0,05 mmHg.

Le spectre RMN est en accord avec la structure de la méthallyloxy-3 aniline.

5.2. *N-(methallyloxy-3 phényl)maléimide:*

Dans un réacteur en verre muni d'une agitation centrale, d'un thermomètre et d'un réfrigérant ascendant maintenu à 50°C sous agitation et dans lequel circule un léger courant d'azote, on introduit simultanément en 20 minutes à l'aide de 2 ampoules de coulée:

— 105 $cm^3$ d'une solution acétonique, contenant 65,2 g de méthallyloxy-3 aniline,

— 105 $cm^3$ d'une solution acétonique, contenant 45,0 g d'anhydride maléique.

La réaction est exothermique et conduit à la formation immédiate d'une suspension jaunâtre.

Lorsque les coulées sont terminées, chaque ampoule est rincée par 10 $cm^3$ d'acétone, qui sont ensuite rajoutés à la masse réactionnelle toujours maintenu sous agitation.

Dans l'ampoule de coulée ayant contenu l'anhydride maléique, on charge 53,0 g d'anhydride acétique et dans l'autre ampoule, on charge 12,2 g de triéthylamine.

Ces deux composés sont alors coulés en 5 minutes dans le réacteur, puis on ajoute 0,8 $cm^3$ d'une solution aqueuse contenant 0,0528 mole d'acétate de nickel pour 100 $cm^3$ de solution.

Le mélange réactionnel est maintenu à reflux sous agitation pendant 2 heures 30 minutes. La température est alors abaissée à 20°C.

Le mélange réactionnel est précipité dans 500 $cm^3$ d'eau distillée à 10°C. L'huile très foncée, visqueuse qui est recueillie est lavée par 250 $cm^3$ de tampon phosphate pH = 7, puis par deux fois 500 $cm^3$ d'eau distillée. Le produit séché sous pression réduite (30 Pa), 15 heures à 30°C est refroidi dans un mélange acétone + carboglace; on obtient ainsi 78 g de solide beige, ayant un point de fusion de 39°C.

Le spectre RMN est en accord avec la structure du N-(méthallyloxy-3 phényl)maléimide.

*Exemple 6*

*Préparation de N-(méthallyloxy-2 phényl)maléimide:*

Ce composé a été préparé à partir de la méthallyloxy-2 aniline.

6.1. *Méthallyloxy-2 aniline:*

Le produit de départ est l'ortho-méthallyloxyacétamidobenzène, dont les conditions d'obtention sont les mêmes que celles décrites dans l'exemple 4, paragraphe 4.1. pour le paraméthallyloxyacétamidobenzène.

Dans un réacteur en verre muni d'une agitation centrale, d'un thermomètre et d'un réfrigérant ascendant, on introduit:

— 205 g d'ortho-méthallyloxyacétamidobenzène

— 400 ml de soude éthanolique 5N

on homogénéise, et on ajoute:

— 13,6 g d'imidazole, soit 20% molaire.

Le mélange réactionnel est maintenu au reflux, sous agitation, pendant 3 heures.

Le mélange réactionnel est refroidi à 20°C.

On élimine l'éthanol sous pression réduite (70 Pa. environ).

On ajoute dans la masse réactionnelle 250 $cm^3$ d'acétate d'éthyle, et on lave par deux fois 250 $cm^3$ d'eau distillée, pour arriver à pH = 7 dans les eaux de lavage.

On élimine l'acétate d'éthyle de la phase organique sous pression réduite (70 Pa. environ) et on obtient 168,10 g de produit brut.

On obtient 136,10 g de produit purifié par distillation à 81°C sous 0,05 mmHg.

Le spectre RMN est en accord avec la structure de la méthallyloxy-2 aniline.

6.2. *N-(méthallyloxy-2 phényl)maléimide:*

Dans un réacteur en verre muni d'une agitation centrale, d'un thermomètre et d'un réfrigérant ascendant maintenu à 50°C sous agitation et dans lequel circule un léger courant d'azote, on introduit simultanément en 20 minutes à l'aide de 2 ampoules de coulée:

— 105 $cm^3$ d'une solution acétonique, contenant 65,2 g de méthallyloxy-2 aniline,

— 105 $cm^3$ d'une solution acétonique, contenant 45,0 g d'anhydride maléique.

La réaction est exothermique et conduit à la formation immédiate d'une suspension jaunâtre.

Lorsque les coulées sont terminées, chaque ampoule est rincée par 10 $cm^3$ d'acétone, qui sont ensuite rajoutés à la masse réactionnelle toujours maintenue sous agitation.

Dans l'ampoule de coulée ayant contenu l'anhydride maléique, on charge 53,0 g d'anhydride acétique et dans l'autre ampoule, on charge 12,2 g de triéthylamine.

Ces deux composés sont alors coulés en 5 minutes dans le réacteur, puis on ajoute 0,8 $cm^3$ d'une solution aqueuse contenant 0,0528 mole d'acétate de nickel pour 100 $cm^3$ de solution.

Le mélange réactionnel est maintenu à reflux sous agitation pendant 2 heures 30 minutes. La température est alors abaissée à 20°C.

Le mélange réactionnel est précipité dans 500 $cm^3$ d'eau distillée glacée sous forte agitation. Le précipité marron est filtré, relavé à l'eau distillée refroidie, et séché pendant 15 heures à 40°C sous pression réduite (30 Pa), et on obtient 70,0 g de précipité beige, ayant un point de fusion de 96°C.

Le spectre RMN est en accord avec la structure du N-(méthallyloxy-2 phényl) maléimide.

*Exemple 7*

On utilise un appareillage constitué par un réacteur en verre, muni d'une agitation centrale et d'une tubulure de dégazage reliée à une pompe à vide par l'intermédiaire de pièges refroidis par un mélange acétone + glace carbonique.

Le réacteur contenant 17,78 g de N-(allyloxy-3 phényl)-maléimide est placé dans un bain d'huile chauffé à 150°C. Après 3 minutes de chauffage, on charge sous agitation, en 3 minutes, 60,44 g de N,N'-4,4'-diphénylméthane-bis-maléimide. Au bout de 2 minutes, la masse réactionnelle est dégazée sous pression réduite (1330 Pa) pendant 10 minutes à 150°C. La masse réactionnelle est alors limpide et elle refroidit à 130°C en 4 minutes.

On rétabli ensuite la pression atmosphérique dans le réacteur et on introduit une solution à base de 0,09 g d'imidazole et de 1,69 g de triallylisocyanurate.

Le mélange est agité quelques instants tout en maintenant la température à 130°C, puis il est dégazé à nouveau sous pression réduite (1330 Pa) pendant 3 minutes.

La pression atmosphérique est rétablie dans le réacteur puis la résine contenue à l'intérieur est coulée dans un moule préchauffé à 120°C et constitué par deux plaques chromées rectangulaires séparés par un entrefer de 4 mm d'épaisseur.

La réticulation de la résine contenue dans le moule est effectuée à pression atmosphérique en étuve chauffée selon le cycle thermique suivant:

— montée en température de 120°C à 150°C en 1 heure,

— maintien à 150°C pendant 1 heure,

— montée en température de 150°C à 200°C en 40 minutes,

— maintien à 200°C pendant 2 heures,

— montée en température de 200°C à 250°C en 40 minutes,

— maintien à 250°C pendant 16 heures,

— refroidissement à température ambiante en 1 heure.

Après démoulage, on obtient une plaque dans laquelle on découpe des éprouvettes de 30 × 7 × 4 mm sur lesquelles on effectue la mesure des caractéristiques initiales de flexion (résistance et module):

. resistance à la flexion:

— à 23°C : 139 MPa

— à 250°C : 73,5 MPa

. module de flexion:

— à 23°C : 3387 MPa

— à 250°C : 2762 MPa.

*Exemple 8*

On utilise un appareillage consitué par un réacteur en verre, muni d'une agitation centrale et d'une tubulure de dégazage reliée à une pompe à vide par l'intermédiaire de pièges refroidis par un mélange glace carbonique + acétone.

Le réacteur est plongé dans un bain d'huile chauffé à 160°C. On charge en trois minutes dans le réacteur sous agitation:

— 90,25 g de N,N'-4,4'-diphénylméthane-bis-maléimide,

— 19,25 g de N-(allyloxy-4 phényl)maléimide,

— 37,50 g de diphénylsilanediol.

Ce mélange fondu et homogène est agité pendant 26 minutes. La masse réactionnelle est refroidie en 10 minutes à 120°C et est dégazée sous pression réduite (660 Pa) pendant 4 minutes.

On rétablit ensuite la pression atmosphérique dans le réacteur et on introduit 2,85 g de triallylisocyanurate contenant 0,15 g d'imidazole.

Le mélange est agité pendant 1 minute tout en maintenant sa température à 120°C, puis il est dégazé à nouveau sous pression réduite (400 Pa) pendant 3 minutes.

La masse réactionnelle est encore agitée pendant 5 minutes à 120°C sous pression atmosphérique, puis est coulée dans un moule préchauffé à 110°C et constitué par deux plaques chromées rectangulaires séparées par un entrefer de 4 mm d'épaisseur.

Sur une autre partie de la composition on détermine:

— l'évolution dans le temps de sa viscosité dynamique à 90°C (mesure à l'aide d'un viscosimètre à mobile tournant),

— son temps de gel à 160°C.

La polymérisation de la résine contenue dans le moule est effectuée à pression atmosphérique, en étuve chauffée selon le cycle thermique suivant:

— montée en température de 110°C à 150°C en 1 heure,

— maintien à 150°C pendant 1 heure,

— montée en température de 150°C à 200°C en 1 heure,

— maintien à 200°C pendant 1 heure.

Au terme de ce cycle, la résine est polymérisée.

On la recuit (toujours dans le moule) pendant 15 h 30 min dans une autre étuve à 250°C. On laisse refroidir à température ambiante.

Après démoulage on obtient une plaque de couleur ocre clair, ne présentant aucun défaut de surface.

Dans cette plaque on découpe des éprouvettes de 30 × 7 × 4 mm sur lesquelles on effectue la mesure des caractéristiques de flexion (résistance et module) avant vieillissement (valeurs initiales) et après 1000 heures à 250°C dans l'air.

Sur ces éprouvettes on mesure également la perte de masse observée après cette période de vieillissement thermique.

Enfin sur une éprouvette broyée en poudre de granulométrie ⩽ 100 µm, on détermine par thermogravimétrie à 5°C/minute sous air, la température de début de décomposition du matériau.

1°) *Les caractéristiques mesurées sur résine non polymérisée sont les suvantes:*

— *Viscosité dynamique à 90°C:*

temps 0: 5,28 Pa × s

après 1 h à 90°C: 12,4 Pa × s

après 2 h à 90°C: 28,8 Pa × s

— *Temps de gel à 160°C:* 10,4 minutes.

2°) *Caractéristiques sur résine polymérisée:*

— *Température de début de décomposition: 344°C*

— *Résistance à la flexion et module de flexion:*

| Résistance à la flexion (en MPa) | | | | Module de flexion (en MPa) | | | |
|---|---|---|---|---|---|---|---|
| initiale | | après 1000 h à 250°C | | initial | | après 1000 h à 250°C | |
| à 23°C | à 250°C | à 23°C | à 250°C | à 23°C | à 250°C | à 23°C | é 250°C |
| 110 | 49 | 91 | 48 | 1950 | 1190 | 2700 | 1700 |

— *perte de masse après 1000 h à 250°C sous air:* 4,58%

— *pas de fissuration* des éprouvettes ainsi vieillies.

*Exemple 9*

On répète l'exemple 8 en remplaçant le N-(allyloxy-4 phényl)maléimide par le N-allyloxy-3 phényl)maléimide.

1°) *Caractéristiques de la résine non polymérisée:*

— *Viscosité initiale à 90°C:* 0,54 Pa × s

— *Temps de gel à 160°C:* 20,9 minutes.

2°) *Caractéristiques de la résine polymérisée:*

— *Température de début de décomposition:* 354°C

— *Résistance à la flexion et module de flexion:*

| Résistance à la flexion (en MPa) | | | | Module de flexion (en MPa) | | | |
|---|---|---|---|---|---|---|---|
| initiale | | après 1000 h à 250° | | initial | | après 1000 h à 250°C | |
| à 23°C | à 250°C | à 23°C | à 250°C | à 23°C | à 250°C | à 23°C | 250°C |
| 100 | 47 | 106 | 56 | 2000 | 1300 | 2500 | 1500 |

— *perte de masse après 1000 h à 250°C sous air:* 5,4%

*Exemple 10*

On opère comme dans l'exemple 8 avec les charges suivantes:

— 85,0 g de N,N'-4,4'-diphénylméthane-bismaléimide,

— 25,0 g de N-(allyloxy-3 phényl)maléimide

— 12,5 g de diphénylsilanediol.

Le mélange fondu est agité pendant 26 minutes.

La masse réactionnelle est refroidie en 10 minutes à 120°C et est dégazée sous 400 Pa pendant 4 minutes.

On rétablit ensuite la pression atmosphérique dans le réacteur et on introduit 2,375 g de triallylisocyanurate contenant 0,125 g d'imidazole.

Le mélange est agité pendant 4 minutes à 135°C, puis il est dégazé à nouveau pendant 12 minutes sous 930 Pa.

La masse réactionnelle ambrée et homogène est coulée dans le moule préchauffé décrit dans l'ensemble 8 et polymérisée comme indiqué dans l'exemple 8.

1°) *Caractéristiques de la résine non polymérisée:*

— *Viscosité initiale à 90°C:* 0,4 Pa × s
— *Temps de gel à 160°C:* 34 minutes.

2°) *Caractéristiques de la résine polymérisée:*

— *Température de début de décomposition:* 358°C
— *Résistance à la flexion et module de flexion:*

| Résistance à la flexion (en MPa) | | | | Module de flexion (en MPa) | | | |
|---|---|---|---|---|---|---|---|
| initiale | | après 1000 h à 250° | | initial | | après 1000 h à 250°C | |
| à 23°C | à 250°C | à 23°C | à 250°C | à 23°C | à 250°C | à 23°C | à 250°C |
| 138 | 76 | 126 | 59 | 3000 | 1900 | 3100 | 2600 |

— *perte de masse après 1000 h à 250°C sous air:* 3,8%.

*Exemple 11*

On opère comme dans l'exemple 8 avec les différents réactifs suivants:

— 90,0 g de N,N'-4,4'-diphénylméthane-bis-maléimide,
— 12,0 g de N,N'-1,3-méthyl-4 phénylène-bis-maléimide,
— 15,0 g de diphénylsilanediol,
— 30,0 g de N-(allyloxy-3 phényl)maléimide,
— 2,85 g de triallylisocyanurate,
— 0,15 g d'imidazole.

1°) *Caractéristiques de la résine non polymérisée:*

— *Viscosité dynamique initiale à 90°C:* 0,82 Pa × s
— *Temps de gel à 160°C:* 30 minutes.

2°) *Caractéristiques de la résine polymérisée:*

— *Résistance à la flexion et module de flexion:*

| Résistance à la flexion (en MPa) | | | | Module de flexion (en MPa) | | | |
|---|---|---|---|---|---|---|---|
| initiale | | après 1000 h à 250°C | | initial | | après 1000 h à 250°C | |
| à 23°C | à 250°C | à 23°C | à 250°C | à 23°C | à 250°C | à 23°C | à 250°C |
| 136 | 74 | 135 | 49 | 3000 | 2100 | 3000 | 2300 |

— *perte de masse après 1000 h à 250°C sous air:* 3,69%

*Exemple 12*

Dans l'appareillage décrit dans l'exemple 8, on charge 18,78 g de N-(allyloxy-3 phényl)maléimide.

On plonge le réacteur dans un bain d'huile chauffé à 160°C et on introduit sous agitation 38,32 g de N,N'-4,4'-diphénylméthane-bis-maléimide et 5,63 g de diphénylsilanediol.

Au but de 8 minutes le mélange est fondu et homogène.

On laisse le mélange sous agitation pendant encore 20 minutes à 160°C, puis on le refroidit à 120°C.

On effectue un dégazage sous une pression de 400 Pa pendant 4 minutes.

On revient à pression atmosphérique et on rajoute un mélange de:

— 0,036 g d'imidazole,
— 1,80 g de N,N',N''-tris(hydroxyéthyl)hexahydrotriazine,
— 2,94 g de phtalate de diallyle.

On dégaze à nouveau sous 1300 Pa pendant 4 minutes à 120°C.

Une partie de la composition est coulée dans le moule décrit dans l'examplè 3 à 110°C. La résine contenue dans le moule est polymérisée selon le cycle thermique indiqué dans l'exemple 3.

1°) *Caractéristiques de la résine non polymérisée:*

— *Viscosité dynamique initiale à 90°C:* 2,57 Pa × s
— *Viscosité dynamique après 2 h à 90°C:* 4,54 Pa × s

2°) *Caractéristiques de la résine polymérisée:*

— Résistance à la flexion initiale à 23°C: 137 MPa

— Résistance à la flexion initiale à 250°C: 53 MPa
— Module de flexion initial à 23°C: 2700 MPa
— Module de flexion initial à 250°C: 1200 MPa

*Exemple 13*

On répète l'exemple 10 en remplaçant le N-(allyloxy-3 phényl)maléimide par le N-(allyloxy-2 phényl)-maléimide.

Les quantités des différents réactifs utilisés sont les suivantes:

— 40,8 g de N,N'-4,4'-diphénylméthane-bis--maléimide,
— 12,0 g de N-(allyloxy-2 phényl)maléimide,
— 6,0 g de diphénylsilanediol,
— 1,14 g de triallylisocyanurate,
— 0,06 g d'imidazole.

La composition obtenue avant polymérisation est limpide.

La polymérisation dans le moule décrit dans l'exemple 8 est effectuée selon le cycle thermique indiqué pour les exemples précédents.

*Caractéristiques de la résine polymérisée:*

— Résistance à la flexion initiale à 23°C: 162 MPa
— Résistance à la flexion initiale à 250°C: 84 MPa
— Module de flexion initial à 23°C: 3100 MPa
— Module de flexion initial à 250°C: 2000 MPa

*Exemple 14*

On utilise un appareillage constitué par un réacteur en verre, muni d'une agitation centrale et d'une tubulure de dégazage reliée à une pompe à vide par l'intermédiaire de pièges refroidis par un mélange glace carbonique + acétone.

Le réacteur est plongé dans un bain d'huile chauffé à 160°C. On charge en trois minutes dans le réacteur sous agitation:

— 67,2 g de N,N'-4,4'-diphénylméthane-bis--maléimide,
— 20,98 g de N-(méthallyloxy-4 phényl)-maléimide,
— 9,82 g de diphénylsilanediol,

Ce mélange fondu et homogène est agité pendant 26 minutes. La masse réactionnelle est refroidi en 10 minutes à 120°C et est dégazée sous pression réduite (660 Pa) pendant 4 minutes.

On rétabli ensuite la pression atmosphérique dans le réacteur et on introduit 2,0 g de triallylisocyanurate contenant 0,10 g d'imidazole.

Le mélange est agité pendant 1 minute tout en maintenant sa température à 120°C, puis il est dégazé à nouveau sous pression réduite (400 Pa) pendant 3 minutes.

La masse réactionnelle est encore agitée pendant 5 minutes à 120°C sous pression atmosphérique, puis est coulée dans un moule préchauffé à 110°C et constitué par deux plaques chromées rectangulaires séparées par un entrefer de 4 mm d'épaisseur.

Sur une autre partie de la composition on détermine:

— l'évolution dans le temps de sa viscosité dynamique à 90°C (mesure à l'aide d'un viscosimètre à mobile tournant),
— son temps de gel à 160°C.

La polymérisation de la résine contenue dans le moule est effectuée à pression atmosphérique, en étuve chauffée selon le cycle thermique suivant:

— montée en température de 100°C à 150°C en 1 heure,
— maintien à 150°C pendant 1 heure,
— montée en température de 150°C à 200°C en 1 heure,
— maintien à 200°C pendant 1 heure.

Au terme de ce cycle, la résine est polymérisée.

On la recuit (toujours dans le moule) pendant 15 h 30 min dans une autre étuve à 250°C. On laisse refroidir à température ambiante. Après démoulage, on obtient une plaque de couleur ocre clair, sans défaut de surface.

Dans cette plaque on découpe des éprouvettes de 30 × 7 × 4 mm sur lesquelles on effectue la mesure des caractéristiques de flexion (résistance et module).

Enfin sur une éprouvette broyée en poudre de granulométrie 100 µm, on détermine par thermogravimétrie à 5°C/minute sous air, la température de début de décomposition du matériau.

1°) *Caractéristiques de la résine non polymérisée:*

— *Viscosité dynamique à 90°C:* temps 0 : 226 Pa × s
— *Viscosité dynamique après 1 h à 90°C:* 386 Pa × s
— *Temps de gel à 160°C:* 13,8 minutes.

2°) *Caractéristiques de la résine polymérisée:*

— *Température de début de décomposition:* 360°C

— *Résistance à la flexion et module de flexion:*
— Résistance à la flexion initiale à 23°C: 113 MPa
à 250°C: 72 MPa
— Module de flexion initiale à 23°C: 2470 MPa
à 250°C: 1820 MPa

*Exemple 15*

On répète l'exemple 14, en remplaçant le N-(méthallyloxy-4 phényl)maléimide par le N-(méthallyloxy-3 phényl)maléimide.

Après démoulage on obtient une plaque de couleur ocre clair.

1°) *Caractéristiques de la résine non polymérisée:*

— *Viscosité dynamique à 90°C:* temps 0 : 7,9 Pa × s
— *Temps de gel à 160°C:* 23,4 minutes

2°) *Caractéristiques de la résine polymérisée:*

— *Température de début de décomposition:* 360°C

— *Résistance à la flexion et module de flexion:*

— Résistance à la flexion initiale à 23°C: 122 MPa
à 250°C: 67 MPa

— Module de flexion initiale à 23°C: 2400 MPa
à 250°C: 1890 MPa

*Exemple 16*

On répète l'exemple 14, en remplaçant le N-(méthallyloxy-4 phényl)maléimide par le N-(méthallyloxy-2 phényl)maléimide.

Après démoulage on obtient une plaque transparente rouge foncé.

1°) *Caractéristiques de la résine non polymérisée:*

— *Viscosité dynamique à 90°C:*
Temps 0 : 8,2 Pa × s
— *Temps de gel à 160°C:* 19,5 minutes

2°) *Caractéristiques de la résine polymérisée:*

— *Température de début de décomposition:* 363°C
— *Résistance à la flexion et module de flexion:*
— Résistance à la flexion initiale à 23°C: 1400 MPa
à 250°C: 67 MPa
— Module de flexion initiale à 23°C: 2530 MPa
à 250°C: 1800 MPa

**Revendications**

1. Nouveaux monomaléimides de formule générale (I):

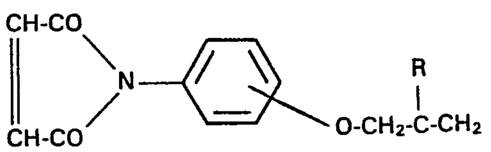

(I)

dans laquelle R représente un atome d'hydrogène ou un radical méthyle.

2. Nouveaux monomaléimides selon la revendication 1 choisis parmi:
— le N-(allyloxy-2 phényl)maléimide,
— le N-(allyloxy-3 phényl)maléimide,
— le N-(allyloxy-4 phényl)maléimide,
— le N-(méthallyloxy-2 phényl)maléimide,
— le N-(méthallyloxy-3 phényl)maléimide,
— le N-(méthallyloxy-4 phényl)maléimide.

3. Nouvelles compositions thermodurcissables, caractérisées en ce qu'elles sont constituées essentiellement par:

A) un prépolymère obtenu par réaction entre 50°C et 300°C de:

a) un bis-imide ou une association de plusieurs bis-imides de formule générale (II):

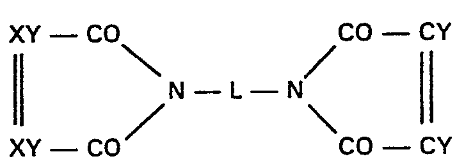

(II)

dans laquelle:

— Y représente un atome d'hydrogène ou un groupement méthyle;

— L représente un radical divalent tel qu'un radical cyclohexylène; un radical phénylène; le radical méthyl-4 phénylène-1,3; le radical méthyl-2 phénylène-1,3; le radical méthyl-5 phénylène-1,3; le radical diéthyl-2,5 méthyl-3 phénylène-1,4 et les radicaux de formule III:

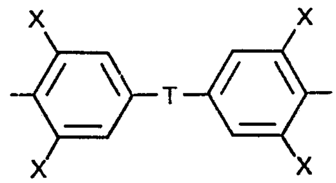

(III)

dans laquelle:

. T représente un lien valentiel simple ou un atome ou groupement suivant:

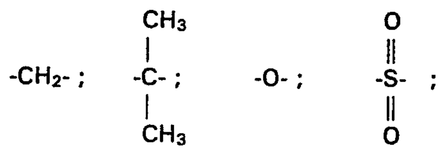

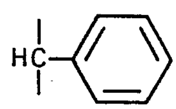 ; 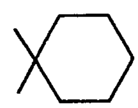

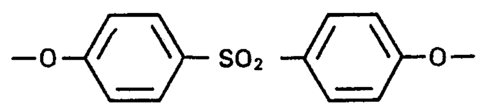

. X représente un atome d'hydrogène, un radical méthyle, éthyle ou isopropyle;
avec

b) un ou plusieurs monomaléimides de formule générale (I):

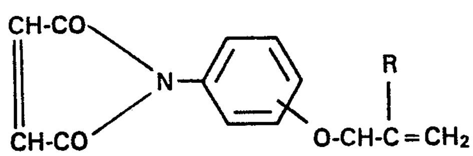

(I)

dans laquelle R représente un atome d'hydrogène ou un radical méthyle;
et éventuellement

c) un composé organosilicique comportant dans sa molécule au moins un groupement hydroxyle lié à un atome de silicium;

et B) de l'imidazole ou un dérivé de l'imidazole.

4. Compositions selon la revendication 3, caractérisées en ce que le bis-maléimide de formule (II) est choisi parmi:
— le N,N'-métaphénylène-bis-maléimide,
— le N,N'-paraphénylène-bis-maléimide,
— le N,N'-4,4'-diphénylméthane-bis-maléimide,
— le N,N'-4,4'-diphényléther-bis-maléimide,
— le N,N'-4,4'-diphénylsulfone-bis-maléimide,

— le N,N'-1,4-cyclohexylène-bis-maléimide,
— le N,N',4,4'-(diphényl-1,1-cyclohexylidène)-bis-maléimide,
— le N,N'-4,4'-(diphényl-2,2 propane)bis-maléimide,
— Le N,N'-4,4'-triphénylméthane-bis-maléimide,
— le N,N'-1,3-méthyl-4 phénylène-bis-maléimide.
— le N,N'-1,3-méthyl-2 phénylène-bis-maléimide.

5. Compositions selon l'une des revendications 3 ou 4, caractérisées en ce que le monomaléimide de formule (I) est choisi parmi:
— le N-(allyloxy-2 phényl)maléimide,
— le N-(allyloxy-3 phényl)maléimide,
— le N-(allyloxy-4 phényl)maléimide,
— le N-(méthallyloxy-2 phényl)maléimide,
— le N-(méthallyloxy-3 phényl)maléimide,
— le N-(méthallyloxy-4 phényl)maléimide,
et leurs mélanges.

6. Compositions selon l'une des revendications 3 à 5, caractérisées en ce que le composé organosilicique, quand on en utilise un, répond à la formule générale (IV):

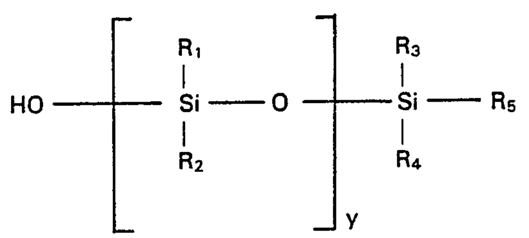
(IV)

dans laquelle $R_1$, $R_2$, $R_3$, $R_4$ et $R_5$, identiques ou différents, représentent:
— un groupement hydroxyle ou un groupement du type $-OR_6$ dans laquelle $R_6$ peut être un radical alkyle, linéaire ou ramifié, ayant de 1 à 6 atomes de carbone ou un radical phényle;
— un atome d'hydrogène;
— un radical alkyle, linéaire ou ramifié, ayant de 1 à 6 atomes de carbone et pouvant être éventuellement substitué par un ou plusieurs atomes de chlore ou de fluor ou par un groupement -CN;
— un radical alcényle, linéaire ou ramifié, ayant de 2 à 6 atomes de carbone;
— un radical phényle, éventuellement substitué par un ou plusieurs radicaux alkyles et/ou alcoxyles ayant de 1 à 4 atomes de carbone, ou par un ou plusieurs atomes de chlore; et y est un nombre entier ou fractionnaire, de 0 à 1000.

7. Compositions selon l'une des revendications 3 à 6, caractérisées en ce que le dérivé imidazole répond à la formule générale (V):

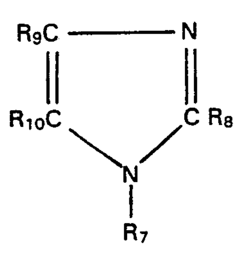
(V)

dans laquelle $R_7$, $R_8$, $R_9$ et $R_{10}$, identiques ou différents, représentent: un atome d'hydrogène, un radical alkyle ou alcoxy ayant de 1 à 20 atomes de carbone, un radical vinyle, un radical phényle, un groupement nitro, $R_9$ pouvant former avec $R_{10}$ et les atomes de carbone auxquels sont liés ces radicaux un cycle unique comme par exemple un cycle benzénique, $R_7$ pouvant également représenter un groupement carbonyle lié à un 2ème cycle imidazole.

8. Compositions selon l'une des revendications 3 à 7, caractérisées en ce que le bis-maléimide utilisé est choisi parmi le N,N'-4,4'-diphénylméthane-bis-maléimide, le N,N'-1,3-méthyl-4 phénylène-bis-maléimide, le N,N'-1,3-méthyl-2 phénylène-bis-maléimide et leurs mélanges.

9. Compositions selon l'une des revendications 3 à 8, caractérisées en ce que le composé organosilicique hydroxylé de formule (IV) qui peut être utilisé est le diphénylsilanediol.

10. Compositions selon l'une des revendications 3 à 5 et 7 et 8, caractérisées en ce qu'elles sont obtenues à partir d'un prépolymère A) préparé à partir d'un ou plusieurs bis-imides de formule (II) et d'un ou de plusieurs monomaléimides de formule (I) avec des quantités de réactifs telles que l'on ait, en poids par rapport au poids total de ces réactifs:
— de 50 à 95% de bis-imide(s) de formule (II),
— et de 5 à 50% de monomaléimide(s) de formule (I).

11. Compositions selon l'une des revendications 3 à 9, caractérisées en ce qu'elles sont obtenues à partir d'un prépolymère A) préparé à partir d'un ou de plusieurs bis-imides de formule (II), d'un ou de plusieurs monomaléimides de formule (I) et d'un composé organosilicique hydroxylé de formule (IV) avec des quantités de réactifs telles que l'on ait, en poids par rapport au poids total de ces réactifs:
— de 40 à 90% de bis-imide(s) de formule (II),
— de 5 à 40% de monomaléimide(s) de formule (I),
— et de 5 à 40% de composé organosilicique hydroxylé de formule (IV).

12. Compositions selon la revendication 11, caractérisées en ce que le polymère A) est préparé à partir de 5 à 20% en poids de composé organosilicique hydroxylé de formule (IV) par rapport au poids total de bis-imide(s), de monomaléimide(s) et de composé organosilicique hydroxylé.

13. Compositions selon l'une des revendications 3 à 12, caractérisées en ce qu'elles contiennent de 0,02 à 1% et de préférence de 0,05 à 0,5% en poids de dérivé imidazole par rapport au poids du prépolymère A).

14. Compositions selon l'une des revendications 3 à 13, caractérisées en ce qu'elles contiennent de 0 à 5% et de préférence de 0,5 à 2% en poids de N,N',N''-tris(hydroxyalkyl)hexahydrotriazine par rapport au prépolymère A).

15. Procédé de préparation de compositions selon l'une des revendications 3 à 14, caractérisé en ce que l'on réalise un mélange intime des composés à groupements maléimides et éventuellement du composé organosilicique hydroxylé, que l'on fond le mélange à une température au plus égale à la température de fusion du maléimide le plus difficile à liquéfier, puis que l'on ajoute le dérivé imidazole, et le cas échéant la N,N',N''-tris-(hydroxyalkyl)hexahydrotriazine, préalablement dissous dans un solvant, sous forte agitation, puis que l'on dégaze et que l'on coule rapidement la résine immédiatement après homogénéisation.

16. Procédé selon la revendication 15, caractérisé en ce que, dans le cas où l'on utilise un composé organosilicique hydroxylé, antérieurement au mélange intime des composés à groupements maléimides et du composé organosilicique hydroxylé, on chauffe ce dernier produit à 150°C environ jusqu'à ce que 40% au moins des groupements hydroxyles initiaux aient disparu.

17. Procédé selon l'une des revendications 15 et 16, caractérisé en ce que le dérivé imidazole, et le cas échéant la N,N',N''-tris(hydroxyalkyl)hexahydrotriazine, sont dissous avant leur addition, dans du triallylisocyanurate, du phtalate de diallyle ou du benzoate d'allyle.

18. Produits conformés préparés à partir des compositions selon l'une des revendications 3 à 14.

**Patetansprüche**

1. Neue Monomaleimide der allgemeinen Formel (I):

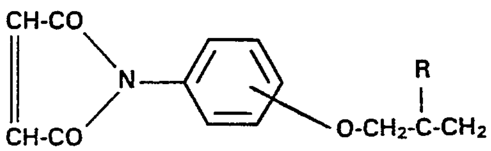

(I)

worin R ein Wasserstoffatom oder einen Methylrest bedeutet.

2. Neue Monomaleimide gemäß Anspruch 1, ausgewählt unter:
— N-(2-Allyloxyphenyl)-maleimid,
— N-(3-Allyloxyphenyl)-maleimid,
— N-(4-Allyloxyphenyl)-maleimid,
— N-(2-Methallyloxyphenyl)-maleimid,
— N-(3-Methallyloxyphenyl)-maleimid,
— N-(4-Methallyloxyphenyl)-maleimid.

3. Neue wärmehärtbare Zusammensetzungen, dadurch gekennzeichnet, daß sie im wesentlichen bestehen aus:

A) einem Prepolymeren, erhalten durch Reaktion zwischen 50°C und 300°C von

a) einem Bisimid oder einer Assoziation mehrerer Bisimide der allgemeinen Formel (II):

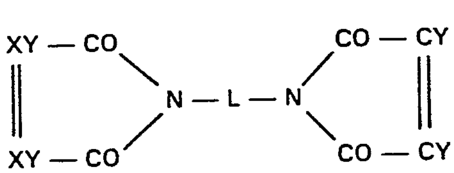

(II)

worin:
— Y ein Wasserstoffatom oder eine Methylgruppe bedeutet;
— L einen zweiwertigen Rest bedeutet wie einen Cyclohexylenrest; einen Phenylenrest; den 4-Methyl-1,3-phenylenrest; den 2-Methyl-1,3-phenylenrest, den 5-Methyl-1,3-phenylenrest; den 2,5-Diethyl-3-methyl-1,4-phenylenrest und die Reste der Formel (III)

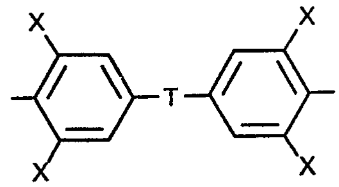

(III)

worin
. T eine Einfachbindung oder ein Atom oder folgende Gruppe bedeutet:

$-CH_2-$ ; 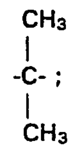 ; -O- ; 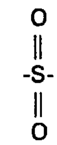 ;

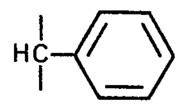 ; 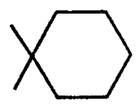

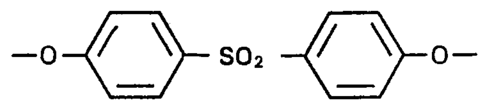

. X ein Wasserstoffatom, einen Methyl-, Ethyl- oder Isopropylrest bedeuet;
mit

b) einem oder mehreren Monomaleimiden der allgemeinen Formel (I)

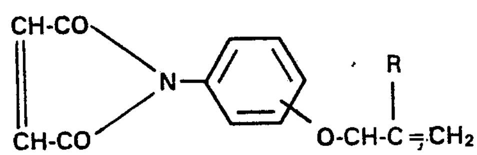

(I)

worin R ein Wasserstoffatom oder einen Methylrest bedeutet; und gegebenenfalls

c) einer Organosiliciumverbindung, die in ihrem Molekül mindestens eine Hydroxylgruppe, gebunden an ein Siliciumatom, trägt;

und B) Imidazol oder einem Derivat des Imidazols.

4. Zusammensetztungen gemäß Anspruch 3, dadurch gekennzeichnet, daß das Bismaleimid der Formel (II) ausgewählt ist unter:
— N,N'-meta-Phenylen-bis-maleimid,
— N,N'-para-Phenylen-bis-maleimid,
— N,N'-4,4'-Diphenylmethan-bis-maleimid,

— N,N'-4,4'-Diphenylether-bis-maleimid,
— N,N'-4,4'-Diphenylsulfon-bis-maleimid,
— N,N'-1,4-Cyclohexylen-bis-maleimid,
— N,N'-4,4'-(1,1-Diphenylcyclohexyliden)-bis-maleimid,
— N,N'-4,4'-(2,2-Diphenylpropan)-bis-maleimid,
— N,N'-4,4'-Triphenylmethan-bis-maleimid,
— N,N'-4-Methyl-1,3-phenylen-bis-maleimid,
— N,N'-2-Methyl-1,3-phenylen-bis-maleimid.

5. Zusammensetzungen gemäß einem der Ansprüche 3 oder 4, dadurch gekennzeichnet, daß das Monomaleimid der Formel (I) ausgewählt ist unter:
— N-(2-Allyloxyphenyl)-maleimid,
— N-(3-Allyloxyphenyl)-maleimid,
— N-(4-Allyloxyphenyl)-maleimid,
— N-(2-Methallyloxyphenyl)-maleimid,
— N-(3-Methallyloxyphenyl)-maleimid,
— N-(4-Methallyloxyphenyl)-maleimid,
und deren Gemischen.

6. Zusammensetzungen gemäß einem der Ansprüche 3 bis 5, dadurch gekennzeichnet, daß die Organosiliciumverbindung, wenn eine solche verwendet wird, der allgemeinen Formel (IV):

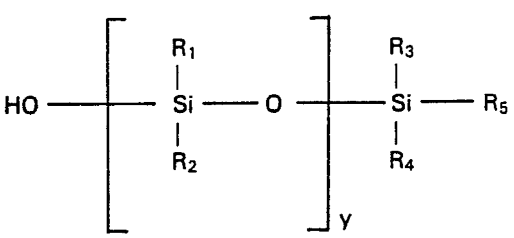 (IV)

entspricht, worin $R_1$, $R_2$, $R_3$, $R_4$ und $R_5$, die identisch oder verschieden sind, bedeuten:
— eine Hydroxylgruppe oder eine Gruppe vom Typ $-OR_6$, worin $R_6$ ein gerader oder verzweigter Alkylrest mit 1 bis 6 Kohlenstoffatomen oder ein Phenylrest sein kann;
— ein Wasserstoffatom;
— eine gerade oder verzweigte Alkylgruppe mit 1 bis 6 Kohlenstoffatomen, die gegebenenfalls durch ein oder mehrere Chlor- oder Fluoratome oder durch eine -CN-Gruppe substituiert sein kann;
— einen geraden oder verzweigten Alkenylrest mit 2 bis 6 Kohlenstoffatomen;
— einen Phenylrest, der gegebenenfalls durch ein oder mehrere Alkyl- und/oder Alkoxyreste mit 1 bis 4 Kohlenstoffatomen oder durch ein oder mehrere Chloratome substituiert ist; und y eine ganze oder Bruchzahl von 0 bis 1000 ist.

7. Zusammensetzungen gemäß einem der Ansprüche 3 bis 6, dadurch gekennzeichnet, daß das Imidazolderivat der allgemeinen Formel (V):

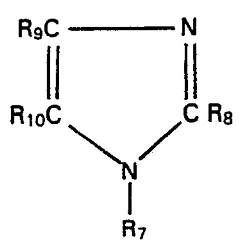 (V)

entspricht, worin $R_7$, $R_8$, $R_9$ und $R_{10}$, die identisch oder verschieden sind, bedeuten: Ein Wasserstoffatom, einen Alkyl- oder Alkoxyrest mit 1 bis 20 Kohlenstoffatomen, einen Vinylrest, einen Phenylrest, eine Nitrogruppe, wobei $R_9$ mit $R_{10}$ und den Kohlenstoffatomen, an die sie gebunden sind, einen Einfachring bilden können wie beispielsweise einen Benzolring, wobei $R_7$ auch eine Carbonylgruppe bedeuten kann, die an einen zweiten Imidazolring gebunden ist.

8. Zusammensetzungen gemäß einem der Ansprüche 3 bis 7, dadurch gekennzeichnet, daß das verwendete Bismaleimid ausgewählt ist unter N,N'-4,4'-Diphenylmethan-bis-maleimid, N,N'-4-Methyl-1,3-phenylen-bis-maleimid, N,N'-2-Methyl-1,3-phenylen-bis-maleimid und deren Gemischen.

9. Zusammensetzungen gemäß einem der Ansprüche 3 bis 8, dadurch gekennzeichnet, daß die hydroxylierte Organosiliciumverbindung der Formel (IV), die verwendet werden kann, Diphenylsilandiol ist.

10. Zusammensetzungen gemäß einem der Ansprüche 3 bis 5 und 7 und 8, dadurch gekennzeichnet, daß sie erhalten werden ausgehend von einem Prepolymeren A), hergestellt ausgehend von einem oder mehreren Bisimiden der Formel (II) und einem oder mehreren Monomaleimiden der Formel (I), mit solchen Mengen an Reaktanten, daß man gewichtsmäßig in bezug auf das Gesamtgewicht dieser Reaktionsteilnehmer hat:
— von 50 bis 95% Bisimid(e) der Formel (II),
— und von 5 bis 50% Monomaleimid(e) der Formel (I).

11. Zusammensetzungen gemäß einem der Ansprüche 3 bis 9, dadurch gekennzeichnet, daß sie erhalten werden ausgehend von einem Prepolymeren A), hergestellt aus einem oder mehreren Bisimiden der Formel (II), einem oder mehreren Monomaleimiden der Formel (I) und einer hydroxylierten Organosiliciumverbindung der Formel (IV), mit solchen Mengen der Reaktionsteilnehmer, daß man gewichtsmäßig in bezug auf das Gesamtgewicht dieser Reaktionsteilnehmer hat:
— 40 bis 90% Bisimid(e) der Formel (II),
— 5 bis 40% Monomaleimid(e) der Formel (I),
— und 5 bis 40% hydroxylierte Organosiliciumverbindung der Formel (IV).

12. Zusammensetzungen gemäß Anspruch 11, dadurch gekennzeichnet, daß das Prepolymere A) hergestellt ist aus 5 bis 20 Gew.-% hydroxylierter Organosiliciumverbindung der Formel (IV) in bezug auf das Gesamtgewicht der Bisimid(e), Monomaleimid(e) und der hydroxylierten Organosiliciumverbindung.

13. Zusammensetzungen gemäß einem der Ansprüche 3 bis 12, dadurch gekennzeichnet, daß sie 0,02 bis 1% und vorzugsweise 0,05 bis 0,5 Gew.-% Imidazolderivat in bezug auf das Gewicht des Prepolymeren A) enthalten.

14. Zusammensetzungen gemäß einem der Ansprüche 3 bis 13, dadurch gekennzeichnet, daß sie 0 bis 5% und vorzugsweise 0,5 bis 2 Gew.-% N,N',N''-Tris-(hydroxyalkyl)-hexahydrotriazin in bezug auf das Prepolymere A) enthalten.

15. Verfahren zur Herstellung der Zusammensetzungen gemäß einem der Ansprüche 3 bis 14, dadurch gekennzeichnet, daß man ein inniges Gemisch der Verbindungen mit den Maleimidgruppen und gegebenenfalls der hydroxylierten Organosiliciumverbindung herstellt, daß man das Gemisch bei einer Temperatur höchsten gleich der Schmelztemperatur des am schwersten zu verflüssigenden Maleimids schmilzt, daß man dann das Imidazolderivat und gegebenenfalls das N,N',N''-Tris-(hydroxyalkyl)-hexahydrotriazin, das vorher in einem Lösungsmittel gelöst wurde, unter starkem Rühren zugibt, daß man dann entgast und daß man das Harz unmittelbar nach seiner Homogenisierung rasch gießt.

16. Verfahren gemäß Anspruch 15, dadurch gekennzeichnet, daß man für den Fall, daß man eine hydroxylierte Organosiliciumverbindung verwendet, vor dem innigen Vermischen der Verbindungen mit Maleimidgruppen und der hydroxylierten organischen Verbindung dieses letztere Produkt auf etwa 150°C erhitzt, bis mindestens 40% der ursprünglichen Hydroxylgruppen verschwunden sind.

17. Verfahren gemäß einem der Ansprüche 15 und 16, dadurch gekennzeichnet daß das Imidazolderivat und gegebenenfalls das N,N'.N''-Tris-(hydroxyalkyl)-hexahydrotriazin vor ihrer Zugabe in Triallylisocyanurat, Diallylphthalat oder Allylbenzoat gelöst werden.

18. Geformte Erzeugnisse, hergestellt aus den Zusammensetzungen gemäß einem der Ansprüche 3 bis 14.

## Claims

1. New monomaleimides of general formula (I):

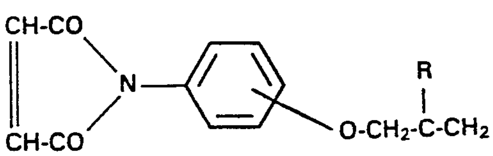

(I)

in which R represents a hydrogen atom or a methyl radical.

2. New monomaleimides according to claim 1, chosen from:
- N-(2-allyloxyphenyl)maleimide,
- N-(3-allyloxyphenyl)maleimide,
- N-(4-allyloxyphenyl)maleimide,
- N-(2-methallyloxyphenyl)maleimide,
- N-(3-methallyloxyphenyl)maleimide,
- N-(4-methallyloxyphenyl)maleimide.

3. New thermosetting compositions, which essentially consist of:

A) a prepolymer obtained by reaction between 50°C and 300°C of:

a) a bis-imide or a combination of several bis-imides of general formula (II):

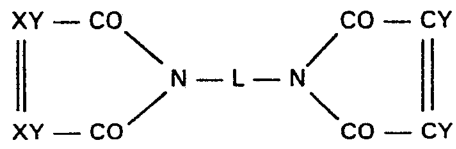

(II)

in which:

— Y represents a hydrogen atom or a methyl group;

— L represents a divalent radical such as a cyclohexylene radical; a phenylene radical; the 4-methyl-1,3-phenylene radical; the 2-methyl-1,3-phenylene radical; the 5-methyl-1,3-phenylene radical; the 2,5-diethyl-3-methyl-1,4-phenylene radical and the radicals of formula (III):

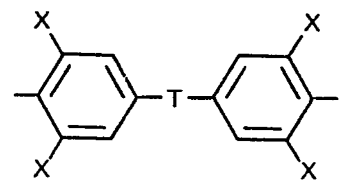

(III)

in which:

T represents a simple valency bond or an atom of the following group:

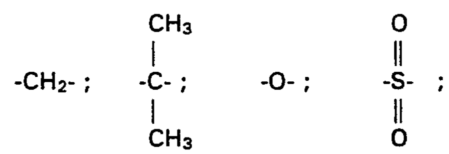

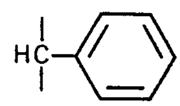 ; 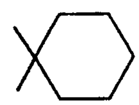

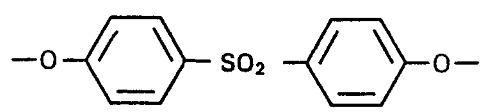

X represents a hydrogen atom, a methyl, ethyl or isopropyl radical;
with

b) one or more monomaleimides of general formula (I):

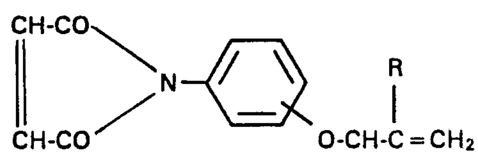

(I)

in which R represents a hydrogen atom or a methyl radical;
and, if appropriate

c) an organosilicic compound containing at least

one hydroxyl group linked to a silicon atom in its molecule;
and

B) imidazole or a derivative of imidazole.

4. Compositions according to claim 3, in which the bis-maleimide of formula (II) is chosen from:

— N,N'-metaphenylene-bis-maleimide,
— N,N'-paraphenylene-bis-maleimide,
— N,N'-4,4'-diphenylmethane-bis-maleimide,
— N,N'-4,4'-diphenylether-bis-maleimide,
— N,N'-4,4'-diphenylsulphone-bis-maleimide,
— N,N'-1,4'-cyclohexylene-bis-maleimide,
— N,N'-4,4'-(1,1-diphenylcyclohexyliden)bis-maleimide,
— N,N'-4,4'-(2,2-diphenylpropane)bis-maleimide,
— N,N'-4,4'-triphenylmethane-bis- maleimide,
— N,N'-1,3-(4-methylphenylene)bis-maleimide,
— N,N'-1,3-(2-methylphenylene)bis-maleimide.

5. Compositions according to either of claims 3 or 4, in which the monomaleimide of formula (I) is chosen from:

— N-(2-allyloxyphenyl)maleimide,
— N-(3-allyloxyphenyl)maleimide,
— N-(4-allyloxyphenyl)maleimide,
— N-(2-methallyloxyphenyl)maleimide,
— N-(3-methallyloxyphenyl)maleimide,
— N-(4-methallyloxyphenyl)maleimide,

and their mixtures.

6. Compositions according to one of claims 3 to 5, in which the organosilicic compound, when one is used, is of the general formula (IV):

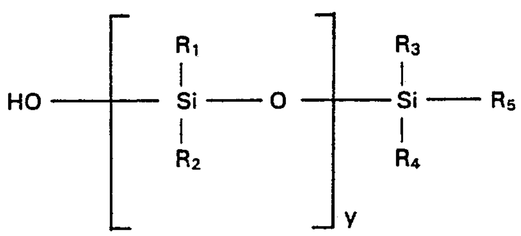
(IV)

in which $R_1$, $R_2$, $R_3$, $R_4$ and $R_5$, which may be identical or different, represent:

— a hydroxyl group or a group of type an $-OR_6$ in which $R_6$ may be a straight-chain or branched alkyl radical containing 1 to 6 carbon atoms or a phenyl radical;
— a hydrogen atom;
— a straight-chain or branched alkyl radical containing 1 to 6 carbon atoms and which may be optionally substituted with one or more chlorine or fluorine atoms or with a -CN group;
— a straight-chain or branched alkenyl radical containing 2 to 6 carbon atoms;
— a phenyl radical, optionally substituted with one or more alkyl and/or alcoxy radicals containing 1 to 4 carbon atoms, or with one or more chlorine atoms; and y is an integer or a fractional number of 0 to 1000.

7. Compositions according to one of Claims 3 to 6, in which the imidazole derivative is of the general formula (V):

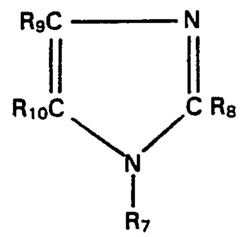
(V)

in which $R_7$, $R_8$, $R_9$ and $R_{10}$, which may be identical or different, represent: a hydrogen atom, an alkyl or alkoxy radical containing 1 to 20 carbon atoms, a vinyl radical, a phenyl radical, a nitro group, $R_9$ being capable of forming with $R_{10}$ and the carbon atoms to which these radicals are linked, a single ring such as, for example, a benzene ring, $R_7$ may also represent a carbonyl group linked to a second imidazole ring.

8. Compositions according to one of claims 3 to 7, in which the bis-maleimide used is chosen from N'N'-4,4'-diphenylmethane-bis-maleimide, N,N'-1,3-(4-methylphenylene)-bis-maleimide, N,N'-1,3-(2-methylphenylene)-bis-maleimide and their mixtures.

9. Compositions according to one of claims 3 to 8, in which the hydroxylated organosilicic compound of formula (IV) which can be used is diphenylsilanediol.

10. Compositions according to one of claims 3 to 5 and 7 and 8, which are obtained from a prepolymer A) prepared from one or more bis-imides of formula (II) and from one or more monomaleimides of formula (I), the quantities of reagents being chosen so as to obtain, by weight in relation to the total weight of these reagents:

— from 50 to 95% of bis-imide(s) of formula (II),
— and from 5 to 50% of monomaleimide(s) of formula (I).

11. Compositions according to one of claims 3 to 9, which are obtained from a prepolymer A) prepared from one or more bis-imides of formula (II), from one or more monomaleimides of formula (I) and from a hydroxylated organosilicic compound of formula (IV), the quantities of reagents being chosen so as to obtain, by weight in relation to the total weight of these reagents:

— from 40 to 90% of bis-imide(s) of formula (II),
— from 5 to 40% of monomaleimide(s) of formula (I),
— and from 5 to 40% of the hydroxylated organosilicic compound of formula (IV).

12. Compositions according to claim 11, in which the prepolymer A is prepared from 5 to 20% (by weight) of the hydroxylated organosilicic compound of formula (IV) relative to the total weight of bis-imide(s), monomaleimide(s) and the hydroxylated organosilicic compound.

13. Compositions according to one of claims 3 to

12, which contain from 0.02 to 1%, and preferably from 0.05 to 0.5%, (by weight) of the imidazole derivative relative to the weight of the prepolymer A).

14. Compositions according to one of claims 3 to 13, which contain from 0 to 5%, and preferably from 0.5 to 2%, (by weight) of N,N',N''-tris(hydroxyalkyl)hexahydrotriazine relative to the prepolymer A).

15. A process for the preparation of compositions according to claims 3 to 14, in which an intimate mixing of the compounds containing maleimide groups and, if appropriate, the hydroxylated organosilicic compound is carried out, in which the mixture is melted at a temperature not exceeding the melting point of the most difficult maleimide to liquefy, in which the imidazole derivative, and where appropriate, the N'N',N''-tris(hydroxyalkyl)-hexahydrotriazine which was previously dissolved in a solvent are then added, with vigorous stirring, in which the mixture is then degassed and in which the resin is rapidly cast immediately after homogenization.

16. A process according to claim 15, in which, in the case where a hydroxylated organosilicic compound is used, prior to the intimate mixing of the compounds containing maleimide groups and the hydroxylated organosilicic compound, this latter product is heated to approximately 150°C until the disappearance of at least 40% of the initial hydroxyl groups.

17. A process according to one of claims 15 and 16, in which, the imidazole derivative, and where appropriate, the N,N',N''-tris(hydroxyalkyl)hexahydrotriazine, are dissolved before their addition, in triallyl isocyanurate, diallyl phthalate or allyl benzoate.

18. Shaped products prepared from the compositions according to one of claims 3 to 14.